# EUROPEAN PATENT SPECIFICATION

(11) **EP 3 023 104 B1**
(45) Date of publication and mention of the grant of the patent: **25.10.2017**
(21) Application number: 14836534.9
(22) Date of filing: 13.06.2014
(51) Int. Cl.: A61K 38/16, A61P 35/00

(54) **RECOMBINANT GANODERMA LUCIDUM IMMUNOMODULATORY PROTEIN (RLZ-8) FOR USE IN TREATING MELANOMA**
REKOMBINANTES GANODERMA-LUCIDUM-IMMUNMODULATORISCHES PROTEIN (RLZ-8) ZUR VERWENDUNG DER BEHANDLUNG VON MELANOM
UNE PROTÉINE IMMUNOMODULATRICE RECOMBINÉE (RZL-8) DE GANODERMA LUCIDUM ET SON UTILISATION POUR TRAITER LE MÉLANOME

(30) Priority: 16.08.2013 CN 201310357176
(43) Date of publication of application: 25.05.2016
(73) Proprietor: Zhang, Xitian, Huangpu District Shanghai 200032 (CN); Sun, Fei, Huangpu District Shanghai 200032 (CN)
(72) Inventor: LIANG, Chongyang, Shanghai 200032 (CN)
(74) Representative: Sun, Yiming
(86) International application number: PCT/CN2014/079813
(87) International publication number: WO 2015/021817

(56) References cited:
- CN-A- 103 417 953
- US-A1- 2007 071 766
- US-A1- 2012 177 674
- C.-T. WU ET AL: "Ling Zhi-8 mediates p53-dependent growth arrest of lung cancer cells proliferation via the ribosomal protein S7-MDM2-p53 pathway", CARCINOGENESIS., vol. 32, no. 12, 1 December 2011 (2011-12-01), pages 1890-1896, XP055245155, GB ISSN: 0143-3334, DOI: 10.1093/carcin/bgr221
- Kohsuke Kino ET AL: "T H E JOURNAL OF BIOLOGICAL CHEMISTRY 0 1989 by The American Society for Biochemistry and Molecular Biology Isolation and Characterization of a New Immunomodulatory Protein, Ling Zhi-8 (LZ-8), from Ganoderrna Lucidiurn*", , 5 January 1989 (1989-01-05), page 472478, XP055304159, Retrieved from the Internet: URL:http://www.jbc.org/content/264/1/472.f ull.pdf [retrieved on 2016-05-21]
- L M HARHAJI TRAJKOVIC ET AL: "Anticancer Properties of Ganoderma Lucidum Methanol Extracts In Vitro and In Vivo", NUTRITION AND CANCER, vol. 61, no. 5, 1 January 2009 (2009-01-01), pages 696-707, XP002762133,
- LI-XIN SUN ET AL: "Ganoderma lucidum polysaccharides antagonize the suppression on lymphocytes induced by culture supernatants of B16F10 melanoma cells", JOURNAL OF PHARMACY AND PHARMACOLOGY, vol. 63, no. 5, 14 April 2011 (2011-04-14) , pages 725-735, XP055163253, ISSN: 0022-3573, DOI: 10.1111/j.2042-7158.2011.01266.x

## Description

### BACKGROUND OF THE PRESENT INVENTION

### Field of Invention

The present invention relates to a field of biomedicine, and more particularly to a use of recombinant *ganoderma* immunoregulatory protein (rLZ-8) in inhibiting growth and metastasis of melanoma.

### Description of Related Arts

Malignant melanoma, also known as Melanoma or Melanosarcoma, is a melanocytes cancer with relatively high malignancy, mostly seen in the skin, and also seen in the mucosa approximate to the skin, such as conjunctiva, oral cavity, nasal cavity, anal canal, rectum, cervix uteri, vagina, penis, and balanus. The European Update of the Guideline on the Diagnosis and Treatment of Melanoma, in 2012, pointed it out that the incidence of the melanoma was increasing, reaching 10-20/10⁵ in Europe, 20-23/10⁵ in United States, and 50-60/10⁵ in Australia. The incidence of the melanoma for the Chinese people with yellow skin is lower than 1/10⁵. According to the statistics of WHO (World Health Organization), around 66,000 people die of the skin cancer every year, wherein 80% die of the melanoma. The advanced melanoma patients have the median survival time of 7-9 months, and the survival rate within a year of merely 25%.

Conventionally, with regard to the treatment method for the orthotopic melanoma, the surgery is preferred for the treatment of the melanoma in situ; the patients with tumor metastasis are mainly treated with the systemic therapy which combines multiple treatments. The systemic therapy for the metastatic melanoma mainly comprises the target therapy, the immune therapy and the chemical therapy. The research related to the target therapy shows the result that, for the patients with the V600E mutation, the target therapy realizing a high-speed tumor response rate (around 50%) does not greatly elongate the lifetime compared to Dacarbazine (DTIC). The immune therapy data indicate that the anti-CTLA-4 antibody (ipilimumab) therapy is the first drug which shows integral survival benefits for the metastatic melanoma, but only has a positive reaction rate of about 10.9%. The chemical drugs belong to the clinical therapy which currently has the longest application history and is most widely applied. Many chemical drugs have equivalent effects, and are applicable to the systemic chemotherapy of the advanced melanoma. US 2012/177674 A1 discloses an isolated and/or purified polypeptide variant or fragment of a fungal immunomodulatory protein which could be obtained from Ganoderma species for use in immunotherapy, treating or preventing cancer due to metastasis or suppression of telomerase activity by down-regulation of the telomerase catalytic subunit (hTERT), or activating natural killer cells, macrophage, increasing serum antibody. US 2007/071766 A1 also discloses the use of Ganoderma immunomodulatory protein for the treatment of melanoma. The chemotherapy is able to cause the tumor shrinkage and the decrease in the symptoms related to the tumor, wherein the DTIC is the most classic drug with the efficiency of merely 5-10%.

As a conclusion, the tumor can be eradicated through surgeries; however, once some individual tumor cell spreads into the body, the surgeries becomes helpless. It brings no good to apply the chemical drugs to the early tumor patients; moreover, the drug resistance of the tumor cells consumes the effectiveness of the chemical drugs in killing the tumor cells and causes merely the toxic side effects. Therefore, the treatment of the malignant melanoma mainly conforms to the usual techniques, namely the surgery, the radiotherapy and the chemotherapy, and the radiotherapy and the chemotherapy again if any deterioration. The chemotherapy is widely applied for the advanced melanoma, and fails to work out in most cases. The non-specific immune therapy seldom generates continuous effects. Therefore, it is urgent to develop a new drug to relieve the symptom of the malignant melanoma patients and elongate the lifetime.

The *ganoderma* immunoregulatory protein (LZ-8) belongs to the family of fungi immunoregulatory proteins. Prior researches prove that the recombinant LZ-8 obtained by gene engineering effectively kills the human gastric cancer cells (SGC-7901) and the human lung carcinoma cells (A549) in vitro. People do not think that the immunoregulatory protein rLZ-8 is able to directly kill the tumor cells in vivo, so there is no report about the anti-tumor-cell level in vivo of the rLZ-8. The eastern always regards the *ganoderma* as the drug for the major difficult diseases, being supreme among the Chinese herbological drugs. As one component of the *ganoderma* rLZ-8, apparently whether the rLZ-8 can be applied for the treatment of human disease or not should be revealed by the anti-cancer in vivo research. In the recent 6 years, the inventor of the present invention researches about the killing effect of rLZ-8 upon nearly hundreds of kinds of tumor cells. It is found that only 10 kinds of tumor cells, comprising the liver cancer cell Hep G2 and the melanoma, are relatively sensitive to the killing effect of the rLZ-8 and show significant difference over the negative contrast group in the aspect of anti-tumor effect, wherein the killing effect upon the melanoma cell is the most significant. The present invention provides a use of the rLZ-8 in preventing and treating growth and metastasis of melanoma, improving survival state and elongating lifetime, and shows surprising effects of the rLZ-8 in killing the melanoma.

### SUMMARY OF THE PRESENT INVENTION

The present invention is related to a use of rLZ-8 as a medicament for treating melanoma with maintaining the leukocyte number during the treatment. A series of experimental means and results show that the rLZ-8 has a significant inhibition effect upon the melanoma, specifically as follows.

The present invention adopts tumor-bearing Kunming mice as research objects, and establishes experimental animal models of mice transplanted with melanoma, respectively a model of orthotopic tumors and a model of metastatic tumors. Five experiment groups, comprising a negative contrast group (physiological saline), a positive contrast group (DTIC), a rLZ-8 low dosage group, a rLZ-8 medium dosage group, and a rLZ-8 high dosage group, are provided in the present invention. Each group comprises 10 mice with melanoma. A dosage of the DTIC is 2.5 mg/kg; dosages in the rLZ-8 low dosage group, the rLZ-8 medium dosage group, and the rLZ-8 high dosage group are respectively 123 µg/kg, 246 µg/kg, and 492 µg/kg. Two administration manners are provided: continuously administrating for 28 days and continuously administrating for 56 days, thereby researching an impact of different drug administration periods on a healing effect. Examination indexes of an anti-tumor pharmacodynamics experiment about the rLZ-8 mainly comprise two aspects of living state and the healing effect. For the aspect of the living state, the tumor-bearing mice are observed from hair glossiness, basic feeding, excrements, movement and agility; for the aspect of the healing effect, mainly a weight, a tumor weight, a tumor volume, a body leukocyte number and a mortality rate are recorded and calculated. In a neutralization test of the rLZ-8, blood serum of normal monkeys (Macaca fascicularis) continuously administrated is selected to test rLZ-8 antibodies by an enzyme-linked immunosorbent assay (ELISA) and test a neutralization activity of the anti-rLZ-8 antibody by a biological activity method. Test results show that the rLZ-8 generates no neutralizing antibody inside the Macaca fascicularis and that the rLZ-8 is not neutralized by antibody throughout the whole melanoma treatment process. In a toxicity test of the rLZ-8, SD rats, as test objects, are intraperitoneally injected with the rLZ-8 protein continuously for 46 days. Test results show that the rLZ-8 protein is able to stimulate growth of the rats; and the rLZ-8 protein generates no apparent adverse impact on liver and kidney function of the rats. In the toxicity test, apparently increased uric acid (UA) plays an important role in an anti-oxidation capacity; the rLZ-8 protein generates no apparent adverse impact on major organs of the rats.

Results of a series of pharmacodynamics experiments of the rLZ-8 upon the experimental animal models of the mice transplanted with melanoma indicate that, the rLZ-8 is able to improve the survival states of the tumor-bearing mice and has significant anti-tumor effect compared with the positive contrast drug DTIC.

With respect to a tumor inhibiting rate and a drug tolerance, the rLZ-8 is able to significantly inhibit the growth of the orthotopic tumors in vivo of the mice. The rLZ-8 high dosage group, continuously administrated for 28 days, has better inhibition effect than the positive contrast group (DTIC). After continuously administrating for 56 days, the rLZ-8 low dosage group, the rLZ-8 medium dosage group and the rLZ-8 high dosage group have higher tumor inhibition rates than the positive contrast group (DTIC), and especially the tumor inhibition rate of the high dosage group is as high as 95.66±1.77%, which is rare in the pharmacodynamics experiments in vivo of the anti-tumor drugs. In the meantime, by comparing data of each group between the administration for the 28 days and the administration for the 56 days, the tumor inhibition rate of the DTIC group decreases, while each rLZ-8 dosage group has an increase of around 15%. It is indicated that the mice start to have a drug tolerance against DTIC during treating the growth of the orthotopic tumors in vivo, while no tolerance emerges in each rLZ-8 dosage group. Therefore, the rLZ-8 is applicable to a long-term administration therapy of the tumors.

With respect to a body immune regulation and safety, the present invention designs a tail vein blood sampling test in a certain experimental period for the tumor-bearing mice. A hematology analyzer detects a leukocyte number of each blood sample. It is found that, the leukocyte number of the tumor-bearing mice of each rLZ-8 dosage group is always at a relatively high level within a normal range, while the leukocyte number of the tumor-bearing mice of the positive contrast group (DTIC) is greatly affected. The leukocyte number of the tumor-bearing mice of the positive contrast group greatly decreases with the treating days increase; the body immunity is severely impaired, and an intense toxicity effect is exhibited. The tail vein blood sampling test shows that the rLZ-8 group is able to maintain the leukocyte number of the body and keep a stability of the body immune system during the process of suppressing the growth and metastasis of the melanoma, which firmly proves a safety of the rLZ-8 in treating the tumor-bearing mice.

With regard to a life elongation rate, a life elongation test of the rLZ-8 on the tumor-bearing mice indicates that the rLZ-8 significantly elongates a lifetime of the tumor-bearing mice compared with the DTIC. By establishing an experimental lung metastasis model of melanoma, it is found that the rLZ-8 significantly inhibits formation and growth of the melanoma lung metastases and has better effect than the positive contrast drug DTIC, with significance.

The present invention has following benefits. The disclosure of the present invention proves that the rLZ-8 is applicable to the preparation of the drug for treating the melanoma. The rLZ-8 is able to kill the melanoma cells in vivo while maintaining the leukocyte number of the body; moreover, the rLZ-8 is able to effectively control the tumor cell metastases towards other tissues, without generating tolerance and with good safety. The above disclosure proves the use of the rLZ-8 in the preparation of the drug for treating the melanoma from multiple levels.

These and other objectives, features, and advantages of the present invention will become apparent from the following detailed description, the accompanying drawings, and the appended claims.

### BRIEF DESCRIPTION OF THE DRAWINGS

Fig. 1 is a diagram of changes in weights of mice, administrated with rLZ-8 for 28 days, according to examples of the present invention.
Fig. 2 is a diagram of the changes in the weights of the mice, administrated with the rLZ-8 for 56 days, according to the examples of the present invention.
Fig. 3 is a curve of body leukocyte numbers of the mice within a treating period according to the examples of the present invention.
Fig. 4 shows a survival rate of each experimental group, after a complete death of a negative contrast group of lung metastasis mice, according to the examples of the present invention.

### DETAILED DESCRIPTION OF THE PREFERRED EMBODIMENT

### Example 1: killing orthotopic melanoma in vivo, maintaining body leukocyte number, and improving living states of tumor-bearing mice, by rLZ-8

### Methods

### (1) Materials and Reagents

Male Kunming mice of 6-8 weeks, weighed 18-22g, were purchased from Laboratory Animal Center of Norman Bethune University of Medical Science, and reared at a specific pathogen-free (SPF) condition in Northeast Normal University, at a temperature controlled at (20 ± 2)°C and a humidity of 48%, and in 12 hours alternating lighting. The mice were transplanted with a melanoma cells line B16-F10.

Dulbecco's modified eagle medium (DMEM), fetal bovine serum, phosphate buffer saline (PBS), trypsin-EDTA, dimethyl sulfoxide (DMSO), 0.9% NaCl solution, Tris-HCl buffer with pH 7.6 for rinsing, 0.05% trypsin, rLZ-8, and DTIC (positive contrast drug).

### (2) Instrument, Equipment and Apparatus

CO₂ thermostat incubator, inverted microscope, pipette, tweezers, clean bench, hematology analyzer, low-speed centrifuge, ultra-low temperature freezing storage cabinet, electronic balance, fresh-keeping cabinet, refrigerator, sterilized pot, water bath pot; mouse raising boxes, water containers; disposable medical sterilized gloves, medical sterilized cotton, 50ml centrifugal tube, pipette tip, cryogenic vial, 10cm cell culture plate, culture flask, 1.5ml EP tube, and cell counting camber; disposable 1ml syringe, mouse bedding, and mouse food.

### (3) Groups and Drug Administration

The mice melanoma cells B16-F10 were cultured in the DMEM containing 10% fetal bovine serum, at 37°C in the CO₂ thermostat incubator. 200µl of B16-F10 cell suspension (containing 1×10⁷ cells) was slowly subcutaneously injected into a dorsal-ventral side of the mouse through 1ml syringe, so as to establish a model of mice transplanted with tumors. After 24 hours, the mice were tail-intravenously injected with the rLZ-8 (123µg/kg, 246µg/kg and 492µg/kg), the DTIC (2.5mg/kg) and the physiological saline respectively. The rLZ-8 was injected once per day; the DTIC was continuously tail-intravenously injected for 5 days and injected for a second time after 3 weeks. During the experiment, living states of the mice were observed; the mice were weighed once every 7 days; blood samples were taken from tail veins of the mice every 2 weeks; volumes of the tumors, subcutaneously injected into the mice, were measured every 2 days; for each group, the tumors were dissected at an end of the experiment and weighed on the balance, with weights recorded. According to a formula that a tumor growth inhibition rate = (an average weight of the tumors of the physiological saline group - an average weight of the tumors of the drug administrated groups) / the average weight of the tumors of the physiological saline group, the inhibition rate of the rLZ-8 upon the growth of the orthotopic tumor was calculated.

### Results

### (1) Living State Analysis Result

After the 28 days of continuously administrating the rLZ-8 to the tumor-bearing mice, no significant difference was observed in aspects of hair glossiness, basic feeding and excrement among the rLZ-8 groups (three dosages), the DTIC group, the physiological saline group and a normal group; the physiological group had poorer movement and agility than the rLZ-8 groups and the DTIC group, and especially the rLZ-8 high dosage group had significantly higher agility than the physiological saline group. After the 56 days of continuously administrating the rLZ-8, it was observed that the rLZ-8 administrated groups had better hair glossiness than the negative contrast group and the DTIC group.

### (2) Analysis Result about Impact of Treatment Drug on Mice Weights

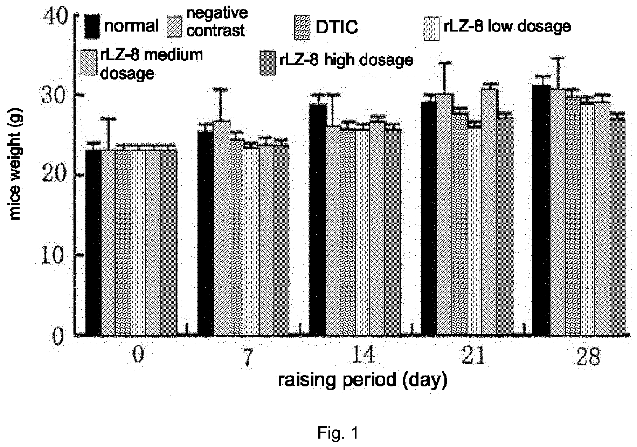

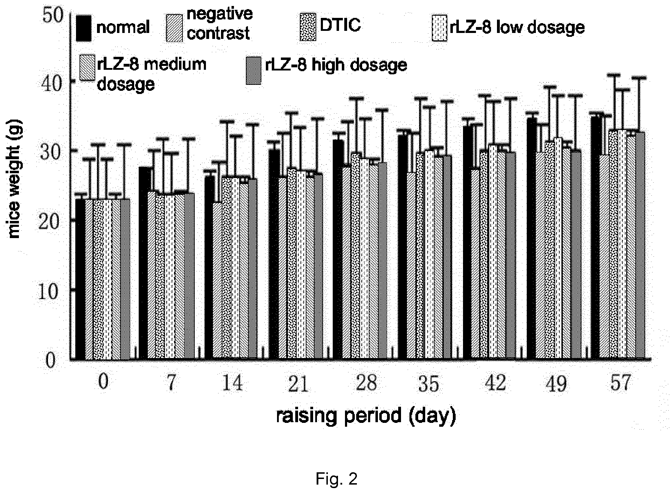

After the 28 days of continuously administrating the rLZ-8 to the tumor-bearing mice, no significant difference existed among the weight of each group before the experiment; after the experiment, the rLZ-8 high dosage group had the slightly smaller weight than the other groups, as showed in Fig. 1. After the 56 days of continuously administrating the rLZ-8, the rLZ-8 groups and the DTIC group had larger weights than the negative contrast group, while the five experiment groups all had the smaller weights than the normal group, as showed in Fig. 2. After the 28 days of continuously administrating and then 28 days of raising without drug administration, for the weights of the finally survived mice, the DTIC group and the rLZ-8 low dosage group had smaller weights than the negative contrast group, and the five experiment groups had the smaller weight than the normal group, which indicated that the rLZ-8 is able to adjust body states of the mice.

### (3) Impact of Treatment Drug on Tumor Morphology of Mice

### Tumor Weight

The tumors were dissected and weighed, and then an average weight of the tumors of each group was calculated. As showed in Table 1 and Table 2, after the 28 days of administrating the rLZ-8, the rLZ-8 high dosage group had the smaller tumor weight than the DTIC group; for the three rLZ-8 groups, with a concentration of the rLZ-8 gradually increased, the weight of the tumor gradually decreased. The rLZ-8 groups had significant difference over the negative contrast group, while the rLZ-8 low dosage group, the rLZ-8 medium dosage group and the rLZ-8 high dosage group had significant difference (n=10, P<0.05). When the drug administration days reach 56 days, by weighing the tumors of the survival mice, the rLZ-8 groups had better tumor inhibition effect than the DTIC group, and especially the rLZ-8 high dosage group had significant difference over the DTIC group (n=10, P<0.05). Therefore, after the 28 days of administrating the rLZ-8, the rLZ-8 high dosage group had smaller tumor weight than the DTIC group; among the three rLZ-8 groups, with the concentration of the rLZ-8 gradually increased, the weight of the subcutaneous tumor gradually decreased; when the days of administration elongated to 56 days, the tumor weights of the survival mice indicated that the rLZ-8 groups had significant tumor inhibition effect over the DTIC group.

### Tumor Volume

For the 28 days of drug administration, with the rLZ-8 concentration gradually increased, the volume of the subcutaneous tumor gradually decreased; the rLZ-8 groups had significantly smaller tumor volumes than the DTIC group, and especially the rLZ-8 groups had significant inhibition on the growth of the tumor compared with the contrast group (n=10). Analyzed from the volumes of the tumors, as showed in Table 1, for the 28 days of the drug administration, the rLZ-8 high dosage group had higher tumor inhibition rate than the DTIC group; among the rLZ-8 groups, with the drug dosage gradually increased, the inhibition rate gradually enhanced. When the days of the drug administration elongated to 56 days, the rLZ-8 groups had particularly significant tumor inhibition rates compared with the DTIC group.

### (4) Test Result of Drug Tolerance Reaction of Body against DTIC and rLZ-8

While inhibiting the weight of the body tumor, the DTIC and the rLZ-8 had a certain impact on the drug tolerance of the body. As showed in Table 1 and Table 2, after administrating the DTIC for 28 days and for 56 days, the inhibition rate greatly decreased, and the tumor growth rate greatly increased, which indicated that the body gradually generated tolerance against the DTIC along with an elongation of a DTIC administration time, so that the tumor inhibition weakened; however, after administrating the rLZ-8 for 28 days and for 56 days, the inhibition rate greatly increased, which indicated that the body tolerance did not increase with an elongation of a rLZ-8 administration time and further proved that the rLZ-8 had healing effect far better than the DTIC.

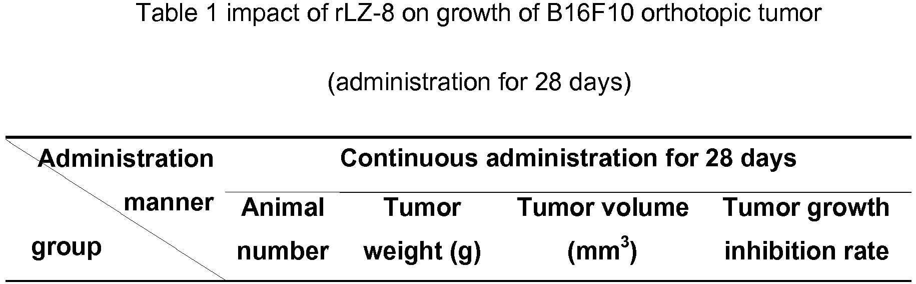

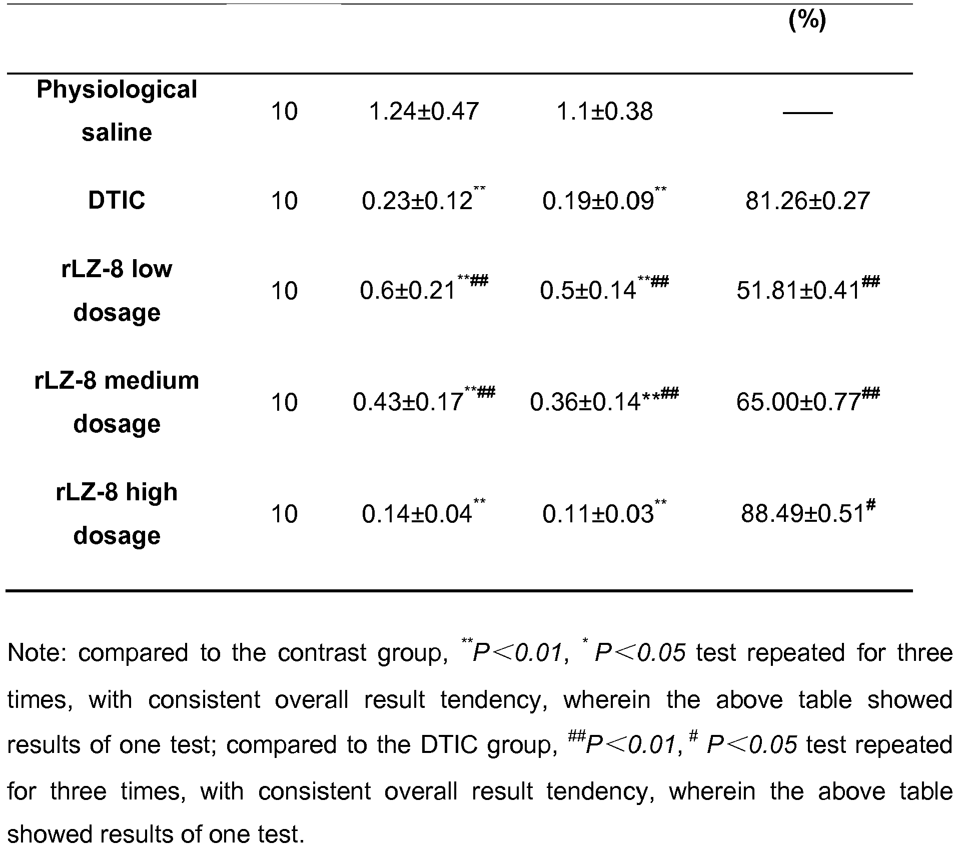

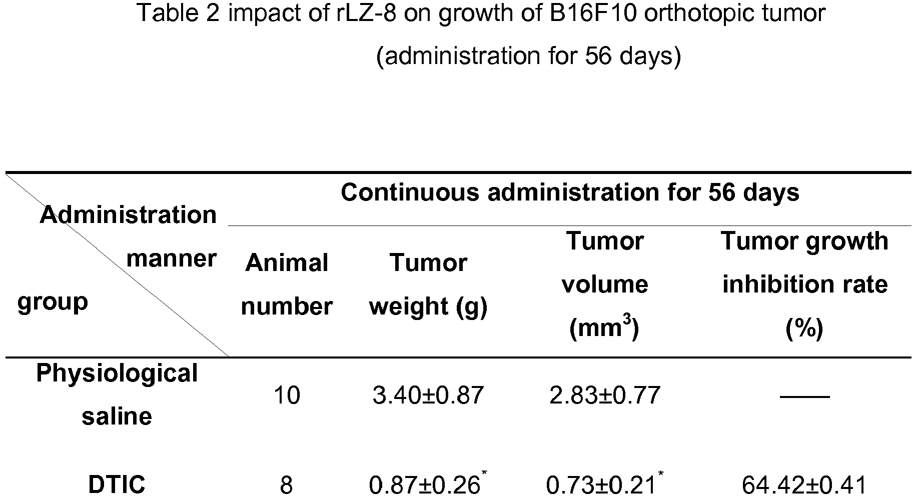

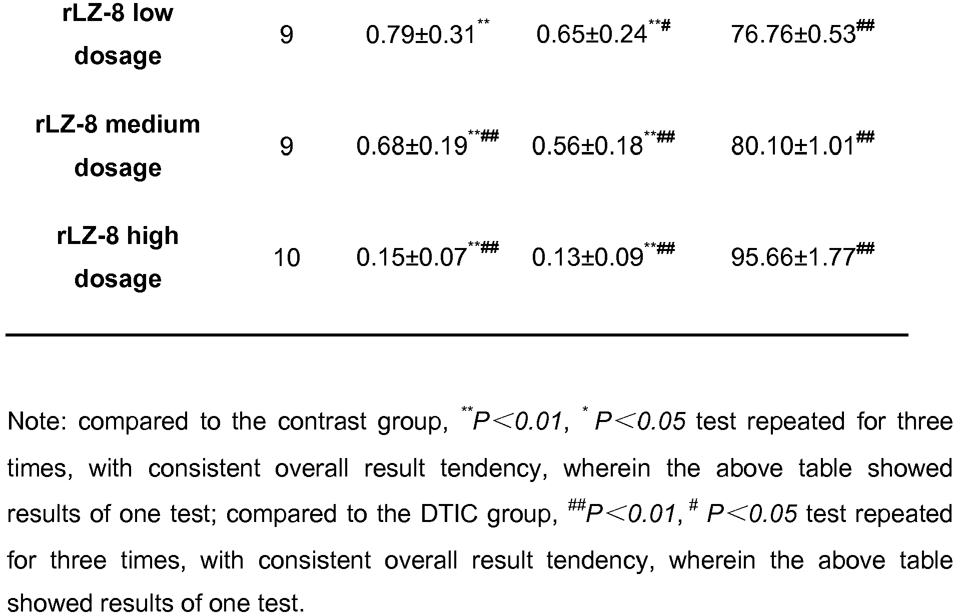

### (5) Impact of rLZ-8 on Body Leukocyte Number and Safety

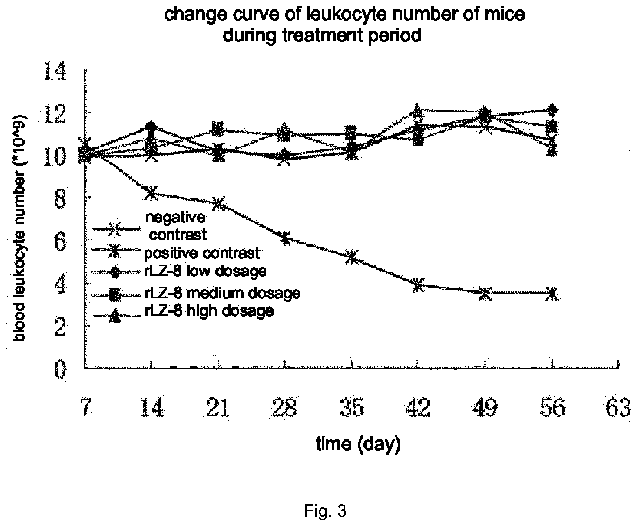

By administrating for 28 days, with the gradually increased rLZ-8 concentration, the body leukocyte number of the mice slightly increased, while the body leukocyte number of the positive drug group (DTIC) evidently decreased; especially each rLZ-8 group had particularly significant impact on the body leukocyte number compared with the contrast group (n=10). By elongating the administration time to 56 days, the leukocyte number of each rLZ-8 group showed significance over the DTIC group (n=10), as showed in Fig. 3; the body leukocyte number of the DTIC group reached a minimal value. By administrating the drug for 28 days and continuing raising without drug administration, the body leukocyte number of the rLZ-8 group slightly decreased; the body leukocyte number of the DTIC group continuously decreased and then showed a stable state for a while. Analyzed from the body leukocyte number, it was showed that the rLZ-8 group exhibited small impact on the body leukocyte number in the anti-tumor treatment experiment, while the positive drug group (DTIC) greatly decreased the body leukocyte number in the anti-tumor treatment experiment, and damaged and weakened the body immune function, causing a hidden danger of immune deficiency in the anti-tumor treatment process. Furthermore, in the anti-tumor experiment, the rLZ-8 not only was able to effectively inhibit the growth of the tumor, but also had functions of maintaining and protecting body immunity. The rLZ-8 had better safety than the positive drug group (DTIC).

### (6) Impact of rLZ-8 on Life Elongation Rate of Tumor-bearing Mice

After continuously administrating the drug for 28 days, no mice died in the experiment period. After continuously administrating the drug for 56 days, death emerged in the negative contrast group and the positive drug group (DTIC), wherein massive death emerged in the positive drug group (DTIC). However, no mice died in each rLZ-8 dosage group, as showed in Table 3. Therefore, the rLZ-8 showed significant difference in maintaining the life elongation rate of the tumor-bearing mice over the DTIC, with significant advantage.

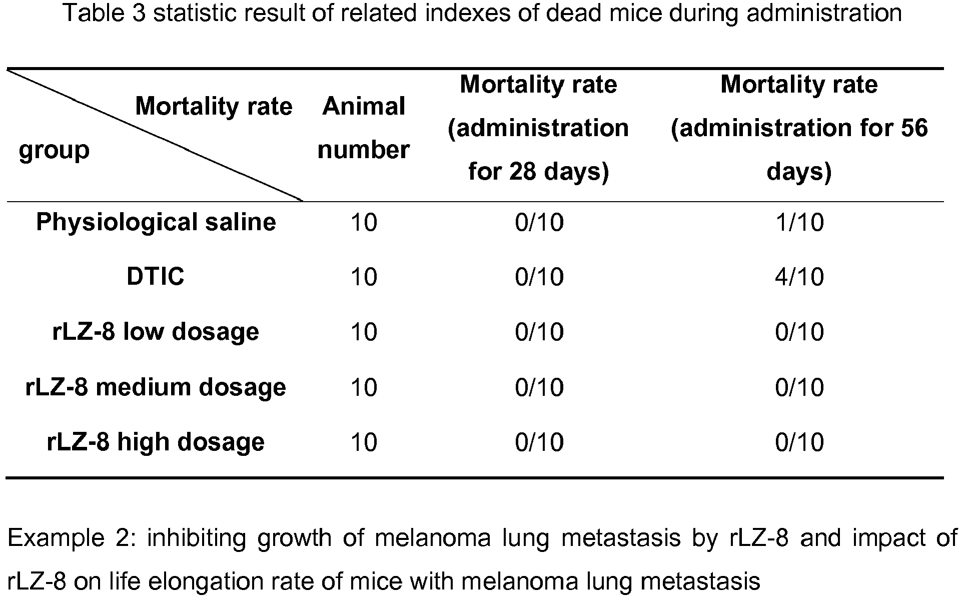

### Methods

### (1) Materials and Reagents

Male Kunming mice of 6-8 weeks, weighed 18-22g, were purchased from Laboratory Animal Center of Norman Bethune University of Medical Science, and reared at an SPF condition in Northeast Normal University, at a temperature controlled at (20 ± 2)°C and a humidity of 48%, and in 12 hours alternating lighting. The mice were transplanted with a melanoma cells line B16-F10. DMEM, fetal bovine serum, PBS, trypsin-EDTA, DMSO, Tris-HCl buffer with pH 7.6 for rinsing, 0.05% trypsin, rLZ-8, and DTIC.

### (2) Instrument, Equipment and Apparatus

### the same with example 1

### (3) Groups and Administration Manner

The mice melanoma cells B16-F10 were cultured in the DMEM containing 10% fetal bovine serum, at 37°C in the CO₂ thermostat incubator. 200µl of B16-F10 melanoma cell suspension (containing 1×10⁷ cells) was slowly intravenously injected into a tail vein of the mouse through 1ml syringe, so as to establish a model of mice transplanted with tumors. After 24 hours, the mice were tail-intravenously injected with the rLZ-8 (123µg/kg, 246µg/kg and 492µg/kg), the DTIC (2.5mg/kg) and the physiological saline respectively. The rLZ-8 was injected once per day; the DTIC was continuously tail-intravenously injected for 5 days and injected for a second time after 3 weeks. After the mice were put to death, lungs of the dead mice were dissected out and the number of black spots on surfaces of the lungs, formed by an aggregation of metastasis cell, was counted, so as to calculate the inhibition rate of the drug against the growth of the lung metastasis as: inhibition rate = (an average metastasis number of the negative contrast group - an average metastasis number of the drug administrated group) / the average metastasis number of the negative contrast group×100%. A time and the number of the dead mouse in each experiment group were recorded in detail, especially during administrating the drug continuously, until the negative contrast group was completely dead; and then, the survival states of the mice in the experiment groups and the contrast groups were analyzed. A survival rate was calculated according to the number of the dead mice in the other groups when all the mice of the negative contrast group were dead, based on a formula that survival rate = (a total number of the mice - the number of the dead mice)/the total number of the mice.

### Results

### (1) Inhibition Rate against Melanoma Lung Metastasis

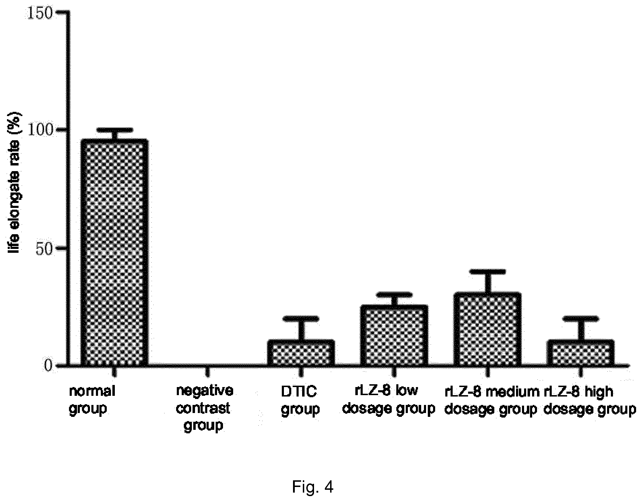

Based on an analysis about the statistics of the melanoma lung metastases, each rLZ-8 dosage group had the smaller number of the tumor metastases than the negative contrast group and the positive contrast group; the positive contrast group had evidently fewer metastases than the negative contrast group. According to the formula of the inhibition rate, the tumor metastasis inhibition rate of the drug in each group was calculated. As showed in Fig. 4, for the 28 days of administrating the drug, the rLZ-8 low dosage group had the inhibition rate of 62.13±1.88% compared with the contrast group; the rLZ-8 medium dosage group had the inhibition rate of 67.65±2.1% compared with the contrast group; and, the rLZ-8 high dosage group had the inhibition rate of 71.17±2.43% compared with the contrast group. Therefore, the rLZ-8 evidently inhibited the formation and the growth of the B16-F10 lung metastases which were intravenously injected into the tail vein of the mice. The positive contrast group had the inhibition rate of 32.04±1.27%, lower than the three experiment drug groups, which indicated that the rLZ-8 had better inhibition effect on the B16-F10 lung metastases which were intravenously injected into the tail vein of the mice, than the positive drug DTIC, as showed in Table 4.

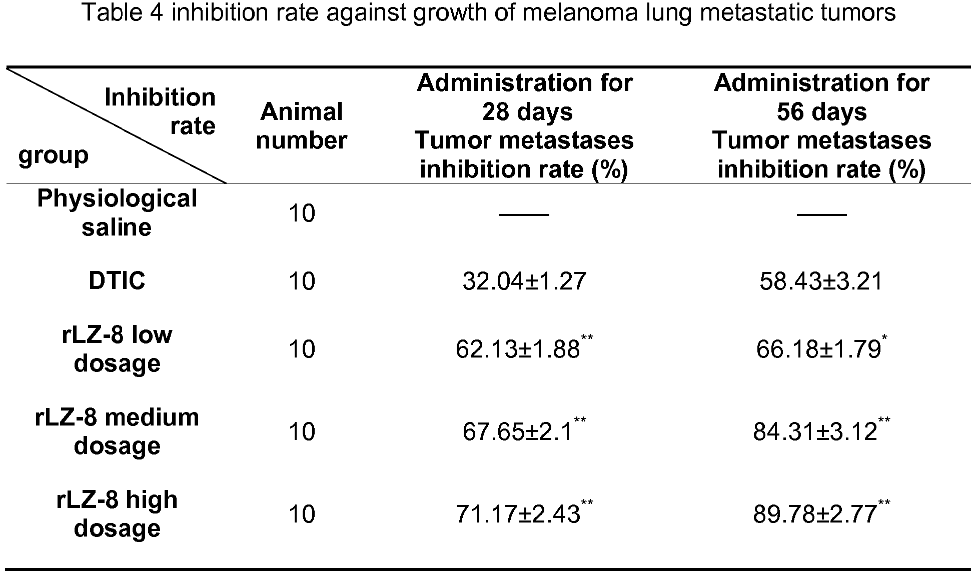

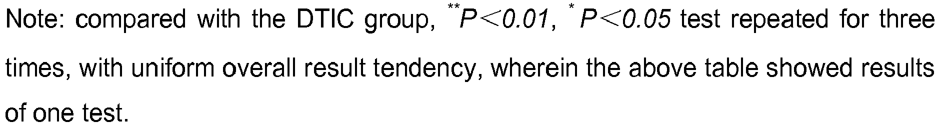

### (2) Impact of rLZ-8 on Life Elongate Rate of Mice with Lung Metastatic Tumors

As showed in Table 4, by recording the time and the number of the dead mouse in each group in detail, the survival states of the mice in the experiment groups and the contrast groups were analyzed. When all the mice of the negative contrast group were dead (respectively on the 87^{th} day and on the 95^{th} day after injecting the tumors, in two repeated tests), the survival rate of the mice remained in the positive drug DTIC group was 10%, namely that 10% of the initial total number of the mice were still alive; the survival rates of the three experiment groups with different concentrations of the experiment drug were respectively 25%, 30% and 10%. Concluded from the results, the positive drug and the experiment drug were effective for an elongation of the survival time of the mice to some extent. Generally speaking, the experiment drug elongated the survival time of the mice and improved the life elongation rate better than the positive drug DTIC.

### Example 3: generation of antibody and neutralizing antibody after continuous multiple administration in vivo of rLZ-8 to Macaca fascicularis

### Methods

As a fungal recombinant genetic engineering drug, it is crucial to track generation of antibody of the rLZ-8 for a preclinical evaluation. Blood serum of normal monkeys, Macaca fascicularis, after being continuously administrated, was selected for testing antibody of the rLZ-8 by ELISA and for testing a neutralization activity of anti-rLZ-8 antibody by a biological activity method.

### Results

On the 9^{th}-11^{th} days of administrating the drug, three monkeys were tested to have low titer (1:5) of the anti-rLZ-8 antibody; after the 28^{th} day of administrating, the titer of the antibody maintained low, within a range of 1:25 to 1:125. No anti-rLZ-8 antibody was tested from the monkeys of the contrast group. Based on a study about impacts of culture media, with the anti-rLZ-8 antibody positive (1:125) monkey blood serum (diluted 10 times) and without the monkey blood serum, on IFN-γ secreting expression stimulated by different concentrations of the rLZ-8, in a cell proliferation curve of the culture medium with the monkey blood serum, a maximum expression value (Emax) of value A decreased to 0.78±0.09; a half maximal inhibitory concentration IC₅₀ increased; and a slope of the curve decreased to 0. 77±0. 20. Therefore, the inhibition effect of the monkey blood serum was not the property of the competitive neutralizing antibody.

Conclusion: the rLZ-8 does not generate the neutralizing antibody in the monkeys and is not neutralized by the antibody during the whole melanoma treatment process.

### Example 4: toxicity test of rLZ-8 on rats

### Methods

### (1) Materials and Reagents

98 SD rats (49 male and 49 female), weighed 100-120g, were purchased from Laboratory Animal Center of Norman Bethune University of Medical Science, and reared at an SPF condition in Northeast Normal University, at a temperature controlled at (20 ± 2)°C and a humidity of 48%, and in 12 hours alternating lighting.

### (2) Instrument, Equipment and Apparatus

### the same with example 1

### (3) Groups and Administration Manner

The administration dosages for the rats were calculated based on the administration dosage for the mice; the rats were divided into a contrast group (physiological saline), a low dosage group (15µl/kg weight), a medium dosage group (30µl/kg weight) and a high dosage group (60µl/kg weight). The administration manner was an intraperitoneal injection.

### (4) Examination Index

Feeding amount, weight; serology: liver function and kidney function; immunity: thymus index and spleen index; serum complements IgM, IgG, C3 and C4; pathology examination: heart, liver, spleen, lung, kidney, pancreas, thymus, gonad etc.

### Results

**Table 5 weight gains statistic data (X̅±s)**

| **group** | **male** | **female** |
|---|---|---|
| contrast | 202.50±13.43 | 124.17±15.16 |
| low dosage | 215.25±17.82 | 140.00±23.48* |
| medium dosage | 218.67±10.89* | 124.08±14.17 |
| high dosage | 221.92±21.84** | 133.37±15.29 |

Compared with the contrast group, ***P<0.01* **P<0.05.*

As showed in Table 5, the rLZ-8 protein medium dosage group and the rLZ-8 protein high dosage group evidently increased the weights of the male rats; the rLZ-8 protein low dosage group evidently increased the weights o the female rats. The rLZ-8 protein had no adverse impact on a general growth state of the SD rats, such as the feeding. Moreover, the rLZ-8 protein medium dosage group and the rLZ-8 protein high dosage group significantly increased the weights of the male rats; the rLZ-8 protein low dosage group significantly increased the weights of the female rats.

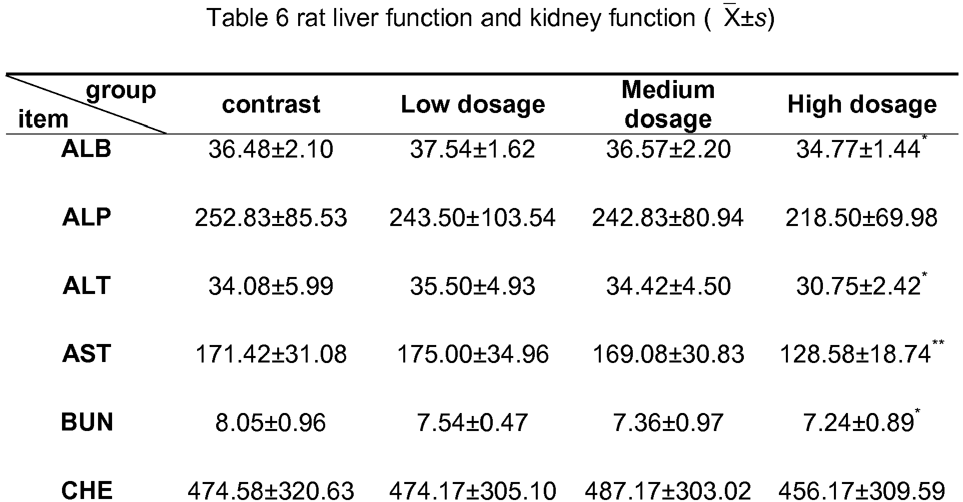

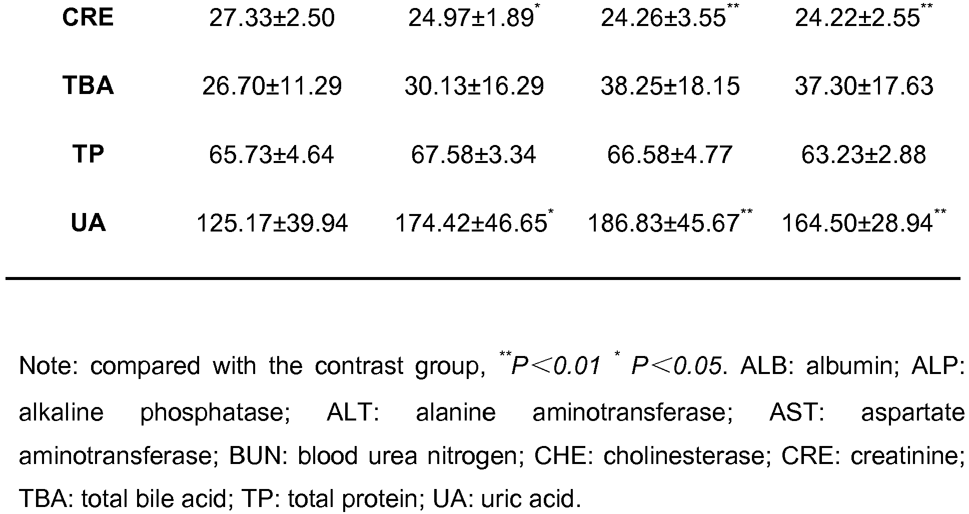

As showed in Table 6, the rLZ-8 protein had no obvious adverse impact on the liver function and the kidney function of the rats. The rLZ-8 low dosage group significantly increased a content of ALB; the rLZ-8 high dosage group had lower content of AST than the rLZ-8 medium dosage group; the rLZ-8 protein groups had lower content of BUN than the contrast group, wherein the rLZ-8 medium dosage group and the rLZ-8 high dosage group had the significantly low content of BUN. The CHE levels of the rLZ-8 low dosage group and the rLZ-8 medium dosage group significantly increased; the CRE level of each rLZ-8 protein group significantly increased. In the rLZ-8 medium dosage group, the TBA level and the UA level significantly increased; in the rLZ-8 low dosage group, the TP level significantly increased. For the female rats, the ALB content and the AST content of the rLZ-8 high dosage group significantly decreased; the TP content of the rLZ-8 high dosage group decreased; and the UA content of each rLZ-8 group significantly increased.

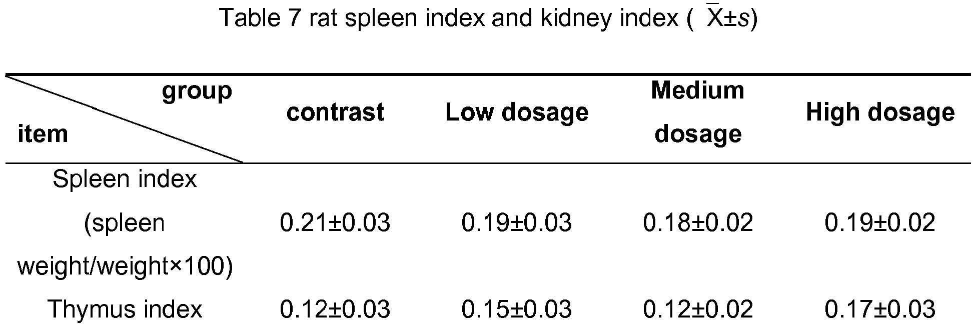

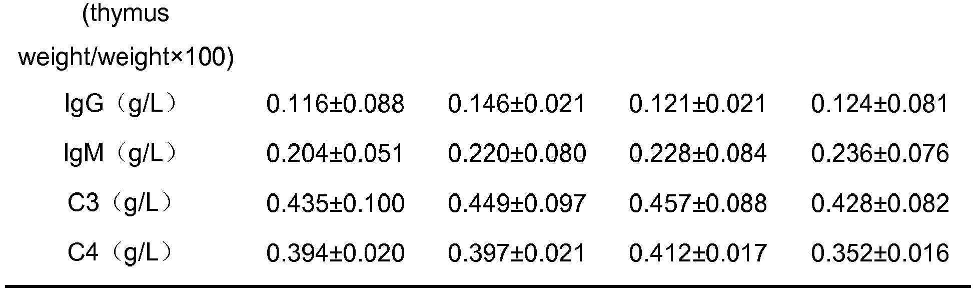

Referring to Table 7, immunity examination results showed that the spleen index (except the low dosage group) and the thymus index of each rLZ-8 group increased compared with the contrast group, but not significantly. With regard to IgG and IgM, each dosage group slightly increased compared to the contrast group, without significance. With regard to C3 and C4, no significant difference existed between each dosage group and the contrast group, and thus, the rLZ-8 had no impact on C3 and C4 of the rats.

Pathology examination: by comparing tested organs with the organs of the contrast group, no obvious morphological change was observed.

### Results

The rLZ-8 protein facilitates the growth of the rats; the rLZ-8 protein has no adverse impact on the liver function and the kidney function of the rats. The UA is beneficial and harmful to the body, wherein the former one refers to an anti-oxidation property and the latter one refers to stimulation to blood vessel smooth muscle cell proliferation and an injury to functions of endothelial cells. In the example 4, the significant increase of UA may play an important role in anti-oxidant capacity; the rLZ-8 protein significantly enhances the immunity of the rats, especially humoral immunity; and, the rLZ-8 protein has no significant adverse impact on major organs of the rats.

### Example 5: rLZ-8 anti-tumor combination and preparation

1. The above pharmacology tests indicate that the anti-tumor effect of the rLZ-8 is significant in maintaining the leukocyte level of the body without toxicity. Therefore, the rLZ-8 is suitable and safe as a drug.
2. As an anti-tumor drug, the rLZ-8 can be administrated orally and parenterally. The administration dosage depends on the symptom, the age, the weight etc. For adults, the oral administration is executed as 10-1000mg per dosage/per person, several times per day; the parenteral administration is executed as 10-100mg, several times per day.
3. A drug for the oral administration of the present invention can be tablets, pills and capsules (hard capsules and soft capsules). The drug for the oral administration comprises the rLZ-8 and at least one inert diluent, such as lactose, mannitol, glucose, starch and polyvinyl pyrrolidone; and further comprises a pharmaceutically acceptable additive, except the inert diluent, such as lubricant, disintegrant and stabilizer. If necessary, the tablets or the pills can be coated with at least one layer of film made of gastric-soluble material or enteric-soluble material. An injection for the parenteral administration of the present invention comprises the rLZ-8 and at least one inert liquid diluent, such as distilled water for injection and physiological saline. The rLZ-8 can be made into lyophilized powder and dissolve into the inert liquid diluent to be injected.

### (1) preparation 1

1000mg of rLZ-8 were dissolved into 100ml of sterilized physiological saline, uniformly mixed, separated into each injection with a concentration of rLZ-8 10mg/ml/per injection to be stored into each bottle, sealed, and sterilized into products. Other items accorded to requirements of the injection under Pharmacopoeia of the People's Republic of China, 2010 edition.

### (2) preparation 2

100g of rLZ-8 and 0.5kg of pharmaceutical starch were prepared into capsules according to known capsule preparation techniques and devices, rLZ-8 10 mg/per capsule. Other items accorded to requirements of the capsule under Pharmacopoeia of the People's Republic of China, 2010 edition.

### (3) preparation 3

100g of rLZ-8, 560g of microcrystalline cellulose, 380g of anhydrous lactose, and 200g of magnesium stearate were prepared into tablets according to known tablet preparation techniques and devices, rLZ-8 10 mg/per tablet. Other items accorded to requirements of the capsule under Pharmacopoeia of the People's Republic of China, 2010 edition.

### (4) preparation 4

A certain amount of the rLZ-8, according to requirements of oral fluid under Pharmacopoeia of the People's Republic of China, 2010 edition, was prepared into the oral fluid through known oral fluid preparation techniques and devices.

## Claims

1. A recombinant *ganoderma* immunoregulatory protein (rLZ-8) for use as a medicament in the treatment of melanoma with maintaining the leukocyte number during the treatment.

2. The recombinant *ganoderma* immunoregulatory protein (rLZ-8) for use according to claim 1, wherein the medicament inhibits a proliferation of the melanoma.

3. The recombinant *ganoderma* immunoregulatory protein (rLZ-8) for use according to claim 1, wherein the medicament inhibits a formation of melanoma metastasis.

4. The recombinant *ganoderma* immunoregulatory protein (rLZ-8) for use according to claim 1, wherein the medicament has core components of a therapeutically effective amount of the rLZ-8 and an arbitrary pharmaceutically acceptable adjuvant.

5. The recombinant *ganoderma* immunoregulatory protein (rLZ-8) for use according to claim 1 or 2, wherein the medicament is administrated orally or non-intestinally, wherein the medicament for being administrated orally is oral fluid, a tablet, a pill or a capsule; and the medicament for being non-intestinally administrated is an external medicine or an injection.

## Patentansprüche

1. Rekombinantes immunregulatorisches *Ganoderma-Protein* (rLZ-8) zur Verwendung als Medikament bei der Behandlung von Melanom mit Erhaltung der Leukozytenzahl während der Behandlung.

2. Rekombinantes immunregulatorisches *Ganoderma-Protein* (rLZ-8) zur Verwendung nach Anspruch 1, wobei das Medikament eine Proliferation des Melanoms hemmt.

3. Rekombinantes immunregulatorisches *Ganoderma-Protein* (rLZ-8) zur Verwendung nach Anspruch 1, wobei das Medikament eine Bildung einer Melanom-Metastasierung hemmt.

4. Rekombinantes immunregulatorisches *Ganoderma-Protein* (rLZ-8) zur Verwendung nach Anspruch 1, wobei das Medikament Kernkomponenten einer therapeutisch wirksamen Menge des rLZ-8 und ein beliebiges pharmazeutisch annehmbares Adjuvans aufweist.

5. Rekombinantes immunregulatorisches *Ganoderma-Protein* (rLZ-8) zur Verwendung nach Anspruch 1 oder 2, wobei das Medikament oral oder nicht-intestinal verabreicht wird, wobei das Medikament für die orale Verabreichung ein orales Fluid, eine Tablette, eine Pille oder eine Kapsel ist; und das Medikament für die nicht-intestinale Verabreichung externe Medizin oder eine Injektion ist.

## Revendications

1. Protéine immunorégulatrice recombinante de *ganoderma* (rLZ-8) destinée à être utilisée en tant que médicament dans le traitement d'un mélanome en maintenant le nombre de leucocytes pendant le traitement.

2. Protéine immunorégulatrice recombinante de *ganoderma* (rLZ-8) destinée à être utilisée selon la revendication 1, dans laquelle le médicament inhibe une prolifération du mélanome.

3. Protéine immunorégulatrice recombinante de *ganoderma* (rLZ-8) destinée à être utilisée selon la revendication 1, dans laquelle le médicament inhibe une formation d'une métastase du mélanome.

4. Protéine immunorégulatrice recombinante de *ganoderma* (rLZ-8) destinée à être utilisée selon la revendication 1, dans laquelle le médicament a des composants de base d'une quantité thérapeutiquement efficace de rLZ-8 et un adjuvant arbitraire pharmaceutiquement acceptable.

5. Protéine immunorégulatrice recombinante de *ganoderma* (rLZ-8) destinée à être utilisée selon la revendication 1 ou 2, dans laquelle le médicament est administré par voie orale ou non intestinale, dans laquelle le médicament destiné à une administration par voie orale est un liquide oral, un comprimé, une pilule ou une gélule ; et le médicament destiné à une administration par voie non intestinale est un médicament externe ou une injection.
